# FASCICULE DE BREVET EUROPEEN

(11) **EP 1 833 555 B1**
(45) Date de publication et mention de la délivrance du brevet: **11.04.2012**
(21) Numéro de dépôt: 05805091.5
(22) Date de dépôt: 31.10.2005
(51) Int. Cl.: A61N 1/34

(54) **DISPOSITIF D'ÉLECTROTHÉRAPIE**
ELEKTROTHERAPIEVORRICHTUNG
ELECTROTHERAPY DEVICE

(30) Priorité: 10.11.2004 FR 0452592
(43) Date de publication de la demande: 19.09.2007
(73) Titulaire: Cosson, Patrick, 83000 Toulon (FR)
(72) Inventeur: Cosson, Patrick, 83000 Toulon (FR)
(74) Mandataire: Nithardt, Roland
(86) Numéro de dépôt international: PCT/IB2005/003239
(87) Numéro de publication internationale: WO 2006/051370

(56) Documents cités:
- US-A- 4 556 064
- US-A- 5 012 816
- US-A- 5 117 826
- US-A- 5 207 231

## Description

La présente invention un dispositif d'électrothérapie comprenant au moins une électrode pour l'application d'un courant de traitement à un patient et un moyen de contrôle comprenant au moins un moyen de déclenchement et agencé pour initier la délivrance d'un profil de courant de traitement prédéterminé dans les différents paramètres dudit courant, notamment l'intensité.

De façon générale, l'électrothérapie est une technique médicale qui utilise les propriétés physiologiques des courants électriques pour traiter certaines affections et, en particulier, elle peut être utilisée dans le traitement des migraines et des maladies apparentées à la migraine.

En effet, la théorie actuellement la plus communément admise pour tenter d'expliquer la physiopathologie des migraines est la théorie trigémino-vasculaire. Cette théorie repose sur l'action conjuguée d'une inflammation neurogène des terminaisons du nerf trijumeau innervant l'hémiface, et de ses conséquences vasculaires (essentiellement une vasodilatation) sur les vaisseaux des méninges innervées par des terminaisons nerveuses provenant de ce même nerf trijumeau.

De façon connue en soi, le nerf trijumeau donne l'innervation sensitive de l'hémiface, comprenant la moitié de la face et du crâne. En ce qui concerne le crâne, il donne, en surface, l'innervation sensitive essentiellement de la région frontale et pariétale en avant, beaucoup moins en région occipitale en arrière. Par contre, il donne également l'innervation des vaisseaux de la base du crâne et d'une grande partie des méninges.

Pour cela, il est composé de fibres sensitives de deux catégories:
- des fibres A 'delta', d'assez gros calibre, myélinisées, et conduisant de ce fait des influx nerveux rapidement. Ces fibres sont chargées de transmettre les douleurs aiguës immédiates précises. Le neurotransmetteur excitateur qu'elles utilisent est le glutamate qui agit rapidement sur ces récepteurs et dont l'effet disparaît en quelques millièmes de secondes. Les terminaisons distales libres de ces fibres sont directement excitées par des phénomènes essentiellement mécaniques (pression, contact) et thermiques. Ces fibres rejoignent, par une chaîne assez directe de trois neurones, le thalamus et les zones d'intégration corticales et sous- corticales. Elles sont responsables de la sensation de douleur immédiate, avec une bonne précision topographique.
- des fibres C, de plus petit calibre, amyéliniques, et conduisant de ce fait plus lentement les influx nerveux. Ces fibres sont chargées de transmettre les douleurs moins immédiates, plus diffuses. Le neurotransmetteur excitateur qu'elles utilisent est, entre autres, la substance P qui agit beaucoup moins rapidement sur ces récepteurs et dont l'effet peut durer plusieurs minutes. Les terminaisons distales libres de ces fibres sont directement excitées par des phénomènes polymodaux de type mécanique (pression, contact), thermique, mais le plus souvent chimique. Ces fibres rejoignent le thalamus et les zones d'intégration corticales et sous-corticales par une chaîne de neurones plus complexe, plus lente, et en étroite connexion avec les structures traversées de la région médullaire haute. À cet endroit, elles rentrent en relation avec les neurones de la substance réticulée, responsable d'un éveil cortical, avec les neurones du raphé médian, de la substance grise périaqueducale, du tectum ventriculaire impliquées dans la régulation endorphinique des phénomènes douloureux. Ces régions sont elles-mêmes sous la domination inhibitrice de neurones issus du système limbique. Elles sont responsables de la sensation de douleur prolongée et chronique, imprécise, diffuse et sourde.

Le nerf trijumeau peut être le siège de "douleurs projetées". Ce phénomène se produit généralement lorsqu'un organe profond est innervé par des collatérales de fibres nerveuses provenant d'un nerf cutané. Dans le cas du nerf trijumeau, des fibres sensitives à point de départ facial cutané ont des collatérales chargées de l'innervation sensitive des vaisseaux de la base du crâne et des méninges. Ainsi un influx nerveux nociceptif prenant naissance au niveau de ces vaisseaux peut être responsable d'une douleur projetée dans le territoire d'innervation cutanée trigéminal de l'hémiface correspondante. Or, il existe des fibres A 'delta' au contact de ces vaisseaux qui sont excitées par la pression due à la vasodilatation de ces vaisseaux. Cette vasodilatation est reconnue comme à l'origine de la douleur migraineuse.

On peut décrire les événements physiopathologiques aboutissant à une crise migraineuse de la façon suivante. Une augmentation de la concentration en sérotonine cérébrale dans certains circuits serait la cause initiale de l'apparition de migraines. Cette augmentation pourrait être génétiquement déterminée chez certaines personne (d'où l'existence de fratries de migraineux). Chez d'autres, elle pourrait être acquise, parfois même sur un terrain prédisposant génétique, à l'occasion des stress répétés de la vie (le plus fréquent semble être l'adolescence de la jeune fille) dont on sait qu'ils ont une nette tendance à élever le niveau de sérotonine dans le système limbique avec toutes les conséquences psychiques connues.

Cette augmentation initiale de sérotonine serait responsable de la vasoconstriction initiale des vaisseaux de la base du crâne et des méninges, facteur d'hypo-perfusion et d'ischémie neuronale se traduisant par les "auras" qui précèdent la douleur migraineuse.

Secondairement, lorsque la sérotonine libère ses récepteurs, une vasodilatation, d'autant plus forte que le niveau de sérotonine impliqué est élevé, s'installe et conduit à une excitation mécanique des fibres nociceptives A 'delta' du nerf trijumeau, responsable de la douleur migraineuse localisée initiale. Cette douleur est projetée également dans le territoire facial du nerf trijumeau par le mécanisme de douleur référée.

Un peu plus tardivement, mais de façon plus diffuse, imprécise et durable, la douleur provient de l'excitation chimique plus lente des fibres C nociceptives, par les substances algogènes libérées par les vaisseaux dilatés par extravasation plasmatique.

Les influx douloureux provenant des fibres C excitent la Substance Grise Péri Aqueducale (SGPA) et le Raphé Médian (RM), structures maintenues en état d'inhibition par le système limbique. Ces deux premières structures libèrent localement des endorphines responsables d'un blocage de l'inhibition du système limbique sur elles-mêmes. Ces structures devraient alors être capables d'activer les circuits inhibiteurs descendants endorphiniques et sérotoninergiques qui bloquent le passage du message douloureux en périphérie, en provenance des fibres C et A'delta'.

Cependant, ceci n'est possible que lorsque les influx incidents sont variables, les neurones négligeant les informations qui durent par phénomène d'accoutumance neuronale. Or la vasodilatation dure, ainsi que ses conséquences mécaniques et surtout chimiques. La SGPA et le RM cessent leur production locale d'endorphine et repassent en état d'inhibition par le système limbique. La crise migraineuse persiste, jusqu'à la réduction lente de la vasodilatation et la disparition des algogènes chimiques. La conséquence directe est une "non-consommation" de la sérotonine, les circuits descendants inhibiteurs sérotoninergiques n'étant plus activés par la SGPA et le RM. D'où l'entrée dans un cercle vicieux qui aggrave progressivement les crises, leur fréquence, leur intensité et leur durée. Indirectement, l'augmentation de la sérotonine dans ces circuits conduit à une augmentation du nombre de récepteurs potentiels à la sérotonine, par le phénomène d'externalisation des récepteurs, dépendant de la concentration en sérotonine circulant dans les vaisseaux, facteur supplémentaire d'aggravation des crises.

L'hypersérotoninergie aggrave la réactivité du système limbique, rendant la personne plus vulnérable aux stress, qui sont eux-mêmes responsables d'une augmentation du niveau général de sérotonine.

L'hyperactivité du système limbique, augmente le blocage par celui-ci de la SGPA et du RM, c'est-à-dire du système endorphinique antalgique naturel, pérennisant ainsi la douleur. Cette explication permet d'aboutir à plusieurs résultats en matière de traitement de la migraine.

D'abord, pour le traitement d'une crise migraineuse, le message douloureux, véhiculé par les fibres A 'delta' puis C, peut être bloqué de plusieurs façons classées en fonction de leur niveau d'action et de leur rapidité d'action :
- une hyperpolarisation des fibres nerveuses par du courant dont le but est de rendre les fibres moins facilement dépolarisables, c'est-à-dire excitables,
- une collision d'influx consistant à bloquer les influx nociceptifs afférents par un courant contraire efférent appliqué sur les mêmes fibres en localisation plus proximale,
- un blocage au niveau du premier relais neuronal de type "Gate Control" utilisant la stimulation de grosses fibres proprioceptives A 'bêta' à faible intensité et fréquence élevée, ces fibres bloquant le passage des influx nociceptifs par inhibition présynaptique des fibres,
- une stimulation des circuits endorphiniques de la SGPA et du RM par excitation des fibres C à forte intensité et faible fréquence.

Ensuite, pour un traitement de fond, il conviendrait de diminuer le niveau de sérotonine. Ceci revient à réactiver le système endorphinique mis au repos par la constance des influx nociceptifs, et par ce moyen, de réactiver les circuits inhibiteurs sérotoninergiques descendants.

Pour cela, il faut provoquer une stimulation électrique des fibres A 'delta' et C sur un mode variable en utilisant des courants de fréquence variable dans le temps. Selon la fréquence, la stimulation porte sur les fibres A 'delta' ou sur les fibres C. Les neurones des structures endorphiniques SGPA et RM ne sont plus le siège d'accoutumance neuronale. Il en résulte une réactivation du système endorphinique et une consommation accrue stabilisatrice de sérotonine.

Les appareils utilisés sont capables de délivrer des courants adéquats dont les caractéristiques sont plus ou moins modulables pour traiter des affections différentes.

En particulier les générateurs de courants permettent de moduler la fréquence des impulsions en Hz, la largeur des impulsions en µs, parfois de moduler l'amplitude et la fréquence de trains d'impulsions de durée, forme et enveloppe variables. Tous ces paramètres peuvent faire l'objet d'une programmation fixe particulière, ce qui évite au praticien ou à l'utilisateur d'avoir à les régler à chaque utilisation spécifique. Cependant, aucun dispositif de l'art antérieur ne propose de profil prédéterminé permettant d'obtenir une variation temporelle de l'intensité du courant puisque celle-ci provoque la douleur du patient si l'intensité est trop élevée.

L'art antérieur connaît déjà des dispositifs d'électrothérapie permettant de s'adapter aux paramètres cutanés des patients. Par exemple, le brevet US 6 650 936 décrit un dispositif permettant le calcul des paramètres électriques d'un patient tels que son impédance. Le signal électrique délivré est alors calculé de façon prédéterminée en fonction de ces paramètres, et par rapport à un patient de référence, afin de maximiser la probabilité de succès du traitement en fonction des paramètres cutanés du patient actuel.

Cependant, un paramètre très important est également réglable, il s'agit de l'intensité de la stimulation en milliampères. Mais ce paramètre ne peut pas être déterminé à l'avance parce qu'il est responsable de la sensation plus ou moins agréable, allant jusqu'à la douleur, que le patient ressent. Or, la sensibilité est un paramètre individuel qui est de ce fait variable d'un individu à l'autre, d'une région du corps à une autre, et qui évolue en fonction de nombreux paramètres physiques et physiologiques comme la résistance cutanée. Celle-ci est également variable en fonction de nombreux paramètres comme le débit sanguin local, la température cutanée. De plus, la résistance cutanée varie au cours de l'établissement du courant lui-même et surtout, la sensibilité à la douleur peut être un paramètre subjectif variant selon les patients et non nécessairement déterminé par des paramètres physiologiques théoriquement pertinents.

C'est pourquoi les appareils existants, ainsi que les traités définissant les conditions d'utilisation des courants ne précisent jamais l'intensité à laquelle l'appareil doit être réglé et se contentent de fournir une indication subjective en rapport avec une comparaison du ressenti avec le seuil de perception du courant, le seuil de sensation déclarée comme douloureuse par le patient considéré. Le praticien règle donc l'intensité en fonction de la réponse orale que donne le patient lorsqu'il augmente progressivement l'intensité.

Le brevet US 4 509 521 divulgue un dispositif d'électrothérapie pour le traitement des maux de tête où l'amplitude du courant doit être à un seuil dépassant 90% du seuil de douleur acceptable par le patient. Celui-ci peut alors régler ce seuil lui- même par un dispositif de réglage. Cependant, le fait que le patient puisse baisser ou augmenter lui-même l'intensité du courant appliqué rend peu efficace le traitement associé, d'autant plus que le traitement proposé est appliqué de façon non programmée et n'est activé que par l'utilisateur ou un opérateur.

L'art antérieur connaît également des dispositifs pour l'application de cycles de traitements préprogrammés. C'est le cas dans la demande publiée sous le N° EP 1 132 110, où un cycle d'intensité est programmé. Ce cycle est par ailleurs adaptable selon les paramètres du patient tel que son impédance. Le dispositif décrit comprend alors un moyen de mesure de l'impédance du patient afin de réguler la tension appliquée en fonction de la valeur de cette impédance.

Cependant, les profils d'intensités préprogrammés de ce document ne sont pas modifiables par l'utilisateur, et, en cas de douleur trop intense, due par exemple à l'application d'une trop forte intensité, l'utilisateur n'a pas la possibilité de modifier le profil. La solution sera donc d'arrêter le programme et de choisir un autre profil moins intense.

L'art antérieur connaît également un dispositif d'électrothérapie par le brevet US 5 207 231. Ce document décrit la possibilité pour l'utilisateur de sélectionner un programme de courant parmi une pluralité de programmes. Cependant, ce dispositif décrit ne permet pas de modifier les programmes sélectionnés en fonction de la douleur du patient pendant l'application du programme.

L'art antérieur connaît également le brevet US 4 556 064 qui concerne un dispositif d'acuponcture électronique qui applique des points d'impulsions aléatoirement au niveau de la peau d'un patient. Cependant, ce dispositif ne permet pas le contrôle du programme par le patient lui-même, par exemple s'il atteint un seuil de douleur trop important.

Le brevet US 5 117 826 a pour objet un appareil médical comprenant un générateur de courant produisant deux types de courant différents :
- un courant à composante continue positive délivré pendant une première phase à visée anti-oedémateuse et cicatrisante ;
- un courant à composante continue nulle délivrée pendant une deuxième phase à visée antalgique.

Cet appareil comprend également un moyen de contrôle pour faire varier l'effet antalgique.

Or, encore une fois, si le moyen de contrôle permet de modifier l'effet antalgique, il ne permet pas le contrôle du courant appliqué, par exemple si le patient a atteint un seuil de douleur trop important.

Enfin, le brevet US 5 012816 décrit un appareil d'acuponcture qui permet d'établir un courant entre une sonde et la main d'un utilisateur.

De façon générale, la difficulté pour apprécier la bonne intensité à appliquer dans le cas d'un traitement par électrothérapie devient totalement prohibitive lorsqu'il s'agit d'utiliser l'électrothérapie dans des affections particulières qui touchent la région de la face et du crâne. C'est le cas pour les migraines, les céphalées, les algies vasculaires de la face, les névralgies du trijumeau, le syndrome d'Arnold, les zonas ophtalmiques et de la face, et autres affections douloureuses de la face et du crâne.

L'objet de la présente invention est donc de remédier aux inconvénients de l'art antérieur concernant la régulation de l'intensité en proposant un dispositif d'électrothérapie permettant l'application d'un profil de courant prédéterminé, tout en tenant compte des seuils de douleur de chaque patient.

Cet objet est atteint par le dispositif tel que défini en préambule et caractérisé en ce que ledit moyen de contrôle comporte en outre des moyens agencés pour modifier le profil du courant de traitement appliqué en fonction du seuil de douleur du patient, lesdits moyens comprenant un moyen d'adaptation limitée dudit courant manipulable par un utilisateur et un moyen de stabilisation agencé pour être activé, ledit moyen de stabilisation, lorsqu'il est activé, provoquant une modification dudit profil de courant de traitement en limitant l'intensité du courant à sa valeur au moment de l'activation dudit moyen de stabilisation.

Dans un mode de réalisation préféré du dispositif, ledit moyen de contrôle comprend un écran d'affichage et au moins une sortie audio.

Par ailleurs, ledit moyen de contrôle comprend en outre un moyen de remise à zéro du courant.

L'art antérieur connaît également le brevet US 4,556,064 (POMERANZ et al.) qui décrit un dispositif d'acuponcture électronique qui applique des points d'impulsions aléatoirement au niveau de la peau d'un patient.

Cependant, le dispositif décrit ne permet pas le contrôle du programme par le patient lui-même, par exemple s'il atteint un seuil de douleur trop important.

L'art antérieur connaît également le brevet US 5,117,826 (BARTELT et al.) qui décrit un appareil médical comprenant un générateur de courant produisant deux types de courant différents :
- un courant à composante continue positive délivré pendant une première phase à visée anti-oedémateuse et cicatrisante ;
- un courant à composante continue nulle délivrée pendant une deuxième phase à visée antalgique.

Il comprend également un moyen de contrôle pour faire varier l'effet antalgique.

Or, encore une fois, si le moyen de contrôle permet de modifier l'effet antalgique, il ne permet pas le contrôle du courant appliqué, par exemple si le patient a atteint un seuil de douleur trop important.

Enfin, l'art antérieur connaît le brevet US 5,012,816, qui décrit un appareil d'acuponcture qui permet d'établir un courant entre une sonde et la main d'un utilisateur.

De façon générale, la difficulté pour apprécier la bonne intensité à appliquer dans le cas d'un traitement par électrothérapie devient totalement prohibitive lorsqu'il s'agit d'utiliser l'électrothérapie dans des affections particulières qui touchent la région de la face et du crâne. C'est le cas pour les Migraines, les Céphalées, les Algies Vasculaires de la Face, les Névralgies du Trijumeau, le Syndrome d'Arnold, les Zonas ophtalmiques et de la face, et autres affections douloureuses de la face et du crâne.

L'objet de la présente invention est donc de remédier aux inconvénients de l'art antérieur concernant la régulation de l'intensité en proposant un dispositif d'électrothérapie permettant l'application d'un profil de courant prédéterminé, tout en tenant compte des seuils de douleur de chaque patient.

Pour ce faire, la présente invention est du type décrit ci-dessus et elle est remarquable, dans son acception la plus large, en ce que elle concerne un dispositif d'électrothérapie, comprenant au moins une électrode pour l'application d'un courant de traitement et un moyen de contrôle, caractérisé en ce que ledit moyen de contrôle comprend au moins un moyen de déclenchement pour initier la délivrance d'un profil de courant prédéterminé dans ses différents paramètres, notamment l'intensité, et un moyen de stabilisation, qui, lorsqu'il est actionné, provoque la modification dudit profil de courant en limitant l'intensité du courant à sa valeur au moment de l'actionnement dudit moyen de stabilisation.

De préférence, ledit moyen de contrôle comprend en outre un moyen d'adaptation limitée du courant manipulable par l'utilisateur.

Avantageusement, ledit moyen de contrôle comprend en outre un écran d'affichage et au moins une sortie audio.

Par ailleurs, ledit moyen de contrôle comprend en outre un moyen de remise à zéro du courant.

Avantageusement, ledit dispositif comprend en outre un moyen de contention pour ladite au moins une électrode.

De préférence, ledit moyen de contention est réglable au moyen d'au moins un ensemble de manchons coulissants.

Enfin, ledit moyen de contention est adaptable à l'utilisation spécifique dudit dispositif d'électrothérapie en fonction de la pathologie à traiter et des variations anatomiques du patient.

L'invention concerne également un procédé de commande dans un dispositif d'électrothérapie comprenant au moins une électrode commandant l'application d'un courant électrique caractérisé en ce qu'il comprend les étapes consistant à :
- délivrer un profil de courant prédéterminé dans ses différents paramètres, notamment l'intensité, au niveau de ladite au moins une électrode ;
- générer un signal provoquant la modification dudit profil de courant en limitant l'intensité du courant à sa valeur au moment de l'actionnement d'un moyen de stabilisation.

Avantageusement, le signal est généré avant ou pendant une phase de croissance du profil prédéterminé de courant.

Par ailleurs, le procédé de commande comprend en outre une possibilité d'adaptation partielle du courant au moment des périodes d'intensité constante dudit profil.

L'invention concerne enfin un procédé de traitement d'un patient utilisant un dispositif d'électrothérapie comprenant au moins une électrode commandant l'application d'un courant électrique caractérisé en ce qu'il comprend les étapes consistant à :
- délivrer un profil de courant d'intensité prédéterminée au niveau de ladite au moins une électrode ;
- générer un signal provoquant la modification dudit profil de courant en limitant l'intensité du courant à sa valeur au moment de l'actionnement d'un moyen de stabilisation, ledit signal étant sensiblement généré lorsque ledit patient a atteint son seuil de douleur acceptable.

Avec un tel procédé de traitement, il est possible de traiter efficacement la migraine d'un patient par électrothérapie à partir d'un profil de courant prédéterminé, sans risque de douleur trop intense pour celui-ci grâce au moyen de stabilisation qu'il peut actionner.

De préférence, ledit profil de courant comprend au moins une première phase comprenant une montée progressive dudit courant depuis une valeur initiale jusqu'à une valeur finale de première phase pendant une période de première phase; une seconde phase comprenant une stabilisation dudit courant à ladite valeur de première phase, et ladite modification étant apte à modifier ladite valeur finale de première phase.

En effet on constate que lors de l'augmentation de l'intensité, le patient ressent une sensation qui va du simple fourmillement à la reproduction assez exacte d'une migraine douloureuse. La sensation devient douloureuse lorsque l'on approche par exemple de 10 milliampères. Si on laisse l'intensité en plateau, la sensation douloureuse disparaît en mois de cinq secondes, et laisse place à une perception non douloureuse du courant qui diminue progressivement en environ 5 minutes.

Si on augmente alors l'intensité, même très faiblement (1 milliampère) le patient perçoit cette augmentation aussi fortement que lorsque l'on a atteint 10 milliampères la première fois. Ceci traduit le fait que le nerf a changé de seuil de sensibilité, hyper polarisé par le courant, et ce seuil se situe désormais juste au-dessus de 10 milliampères. Une petite augmentation de 1 milliampère seulement dépasse le seuil d'excitabilité et la sensation est à nouveau maximale jusqu'à la prochaine adaptation du nerf.

Pendant cette phase, les caractéristiques précises du courant sont déterminées par le logiciel de sorte qu'une stimulation des fibres A 'delta', C et A 'bêta' soit obtenue. De plus, le logiciel met en oeuvre une variabilité des caractéristiques de stimulation de telle sorte que la stimulation de ces différentes fibres ne soit pas constante. Ce mécanisme assure une stimulation permanente du système endorphinique et des circuits inhibiteurs sérotoninergiques descendants, sans effet d'Accoutumance Neuronale (Cette remarque est valable pour toutes les phases).

De préférence, ledit profil de courant comprend en outre au moins une troisième phase comprenant une montée progressive dudit courant depuis ladite valeur de première phase jusqu'à une valeur finale de seconde phase pendant une période de seconde phase; une troisième phase comprenant une stabilisation dudit courant à ladite valeur finale de seconde phase, et ladite modification étant apte à modifier ladite valeur finale de seconde phase.

Selon un profil préféré de l'invention, le profil de courant prédéfini augmente lors d'une première phase depuis la valeur nulle jusqu'à la valeur de 10 mA à la vitesse de 1mA/s, puis, il se stabilise pendant 5 minutes. Puis, il augmente de 1mA pendant une seconde à la vitesse de 1mA/s. puis il se stabilise pendant 5 minutes. Ces dernières augmentation et stabilisation sont alors itérées quatre fois au total. Lors d'une dernière phase, le courant augmente encore une fois de 1mA pendant une seconde à la vitesse de 1mA/s, puis est ramené à 0 mA.

Lors des montées de courant, le moyen de stabilisation permet alors de limiter la montée progressive du courant si le patient trouve la sensation trop désagréable.

On comprendra mieux l'invention à l'aide de la description, faite ci-après à titre purement explicatif, d'un mode de réalisation de l'invention, en référence aux figures annexées où :
- la figure 1 est une représentation du positionnement des électrodes délivrant le courant de traitement ;
- la figure 2 représente le dispositif de contrôle selon l'invention ;
- la figure 3 représente le profil de courant prédéterminé dépendant du contrôle automatique par le dispositif original et une indication corrélative du ressenti du patient ;
- la figure 4 est un diagramme bloc du dispositif selon l'invention.

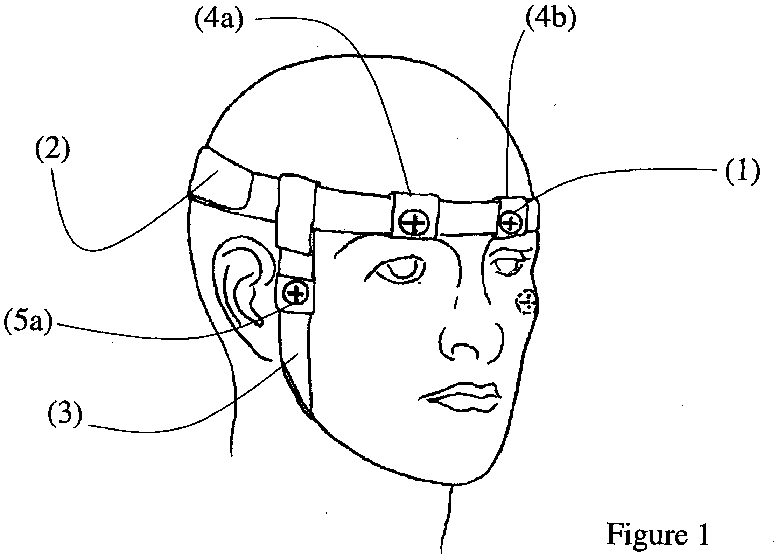

Illustré figure 1, le dispositif comprend un système de maintien et de placement des électrodes. II utilise des électrodes classiques adhérentes interchangeables (1).

Ces électrodes sont placées sur un support spécifique qui va assurer à la fois leur maintien en bonne position et leur positionnement adéquat à la manière d'un gabarit ajustable.

Le support reçoit quatre électrodes associées deux à deux latéralement, chaque voie disposant d'une électrode frontale sus-orbitaire et d'une électrode zygomatique. Cette disposition correspond à la projection en surface de la trifurcation du nerf trijumeau au niveau zygomatique et du passage sur l'arcade sourcilière de sa branche ophtalmique.

Le support est composé d'un bandeau 2 et d'une jugulaire réglables 3.

Le bandeau 2 est un bandeau en tissus ajustable large et plat se disposant comme un serre-tête sur le front et les tempes de sorte qu'il soit horizontal avec son bord inférieur aligné au bord supérieur des sourcils.

Ce bandeau est équipé de deux petits manchons 4a et 4b coulissant sur le bandeau qui vont être placés à l'union du tiers interne et des deux tiers externes de chaque arcade sourcilière.

La face plate en regard de la peau de ces manchons comporte une surface adhérente sur laquelle vient se coller la face non active de l'électrode adhérente, de façon à positionner cette dernière sur la peau.

Le support comprend également une jugulaire 3 qui se fixe sur le bandeau de chaque coté de la tête en passant en avant de l'oreille verticalement.

Sa taille est ajustée en réglant de chaque côté sa longueur.

Elle comporte également les mêmes manchons 5a coulissants adhérents pour porter les électrodes.

Ceux-ci sont ajustés pour venir en regard de la dépression se situant 1 cm en avant de l'oreille sur la ligne horizontale partant du conduit auditif, en dessous de l'apophyse zygomatique.

Ce support comprend donc un gabarit de placement automatique des électrodes permettant une adaptation particulièrement avantageuse aux spécificités morphologiques particulières à chaque patient.

Par ailleurs, le courant délivré dans les électrodes incluses dans le support possède des caractéristiques spécifiques adaptées à la pathologie à traiter. Le praticien est apte à déterminer ces paramètres préprogrammés selon la pathologie.

Le courant délivré est issu de préférence de deux canaux permettant chacun d'alimenter une paire d'électrodes. L'impulsion est de préférence de type rectangulaire. Il faut par ailleurs noter que la randomisation de la fréquence est nécessaire afin que les nerfs ne s'accoutument pas à une valeur constante de la fréquence d'excitation.

Les courants délivrés sur les deux canaux de l'appareil sont régulés à la fois automatiquement par une logique interne, et par une action manuelle de l'utilisateur. Le dispositif de contrôle est alors spécifiquement adapté à un tel fonctionnement.

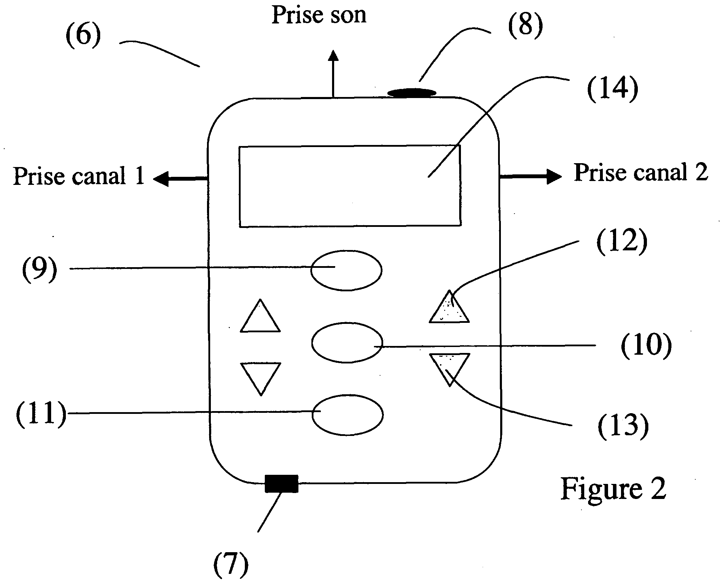

Illustré figure 2, ce dispositif 6 comprend un bouton marche/arrêt 7, un bouton de réglage de volume sonore 8, deux prises mini-jack pour les canaux 1 et 2, une prise mini-jack pour la sortie sonore, 3 boutons de contrôle 9, 10, 11, des boutons triangulaires 12 d'augmentation des canaux 1 et 2, des boutons triangulaires 13 de diminution des canaux 1 et 2 et un écran à cristaux liquides 14.

Pour des raisons de sécurité, l'appareil est programmé pour ne pas pouvoir délivrer un courant supérieur à une valeur limite compatible avec la pathologie à traiter.

Une des caractéristiques importante du dispositif selon l'invention est la présence des boutons 9, 10, 11 du moyen de contrôle permettant à l'utilisateur de contrôler l'augmentation du courant en fonction de son seuil de douleur. En particulier, un des trois boutons, par exemple le bouton central 10 permet de figer l'intensité à sa valeur courante, lorsque le patient considère que son seuil de douleur est atteint. Un autre bouton, par exemple le bouton supérieur 9 déclenche la montée progressive du courant, alors que le dernier bouton, par exemple le bouton inférieur 11 permet le retour de l'intensité à 0.

Il est entendu que ces boutons peuvent être identifiables par des couleurs différentes. Afin de faciliter la description de la présente invention, on considérera donc que le bouton de déclenchement de la délivrance du profil d'intensité est un bouton vert, que le bouton de stabilisation de l'intensité est le bouton orange, et que le bouton de remise à zéro de l'intensité est le bouton rouge.

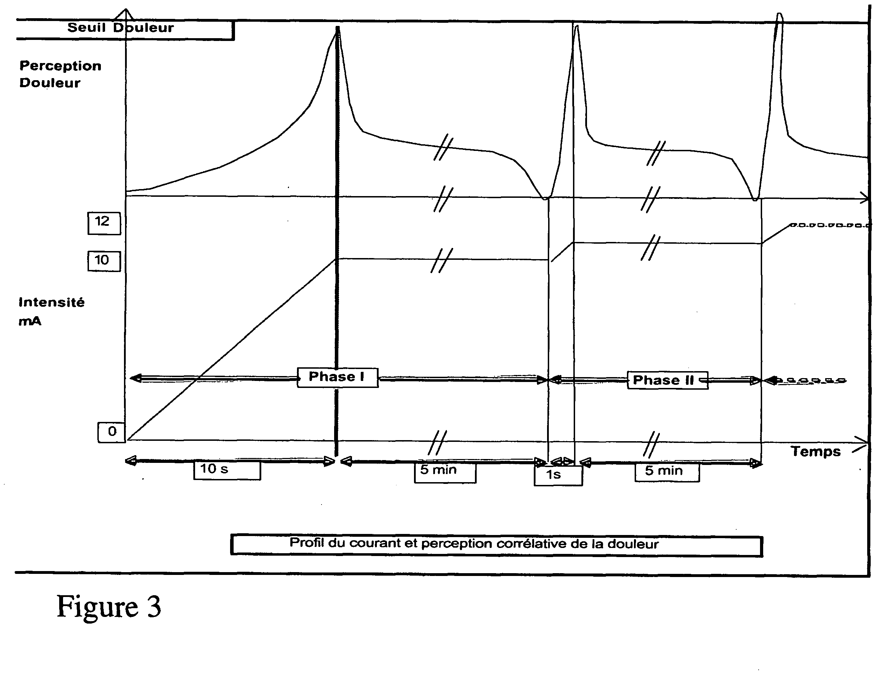

Nous décrivons maintenant un mode d'utilisation du dispositif selon l'invention lors de son fonctionnement pour le traitement par exemple des migraines. Ce mode d'utilisation se caractérise par un profil de courant, d'amplitude régulée et prédéterminée, appliqué au niveau des électrodes comme illustré figure 3, ce courant étant contrôlé d'une part par un programme interne avec base de temps inséré dans le dispositif de contrôle 6 et d'autre part par l'utilisateur grâce aux boutons du dispositif de contrôle.

De préférence, lorsque le bouton marche/arrêt 7 est mis sur marche, ceci déclenche l'émission d'un ensemble de phrases explicatives ou d'informations sonores et/ou visuelles. Il est entendu que ce principe d'émission sonore par la sortie son ou d'émission visuelle sur l'écran du dispositif n'est utilisé que pour faciliter l'utilisation du dispositif par un utilisateur et qu'il peut être omis dans un mode de réalisation particulier de l'invention.

Lors d'une première phase d'utilisation l'utilisateur appuie sur le bouton vert et déclenche la montée progressive de l'intensité sur le canal 1, par exemple de 0 à 10 mA, à la vitesse de par exemple 1 mA par seconde pendant par exemple 10 secondes. Si l'on appuie sur le bouton orange lors de la montée de l'intensité, celle-ci se fige à sa valeur courante. L'actionnement du bouton vert de déclenchement initie donc l'application du profil prédéterminé.

Lorsque les par exemple dix secondes sont atteintes ou lorsque le bouton orange est pressé en cours de montée, une montée progressive de l'intensité sur le canal 2 est réalisée de la même façon et avec la même possibilité de régulation.

Lorsque la montée en intensité du canal 2 est terminée (au bout des par exemple dix secondes de montée ou lorsque le bouton orange est pressé), le système est programmé pour maintenir le courant délivré dans chacun des canaux pendant une durée de stabilisation programmée de par exemple 4 minutes et 45 secondes.

Au bout des premières secondes de ce palier, une phrase est par exemple prononcée par la sortie sonore et visualisée sur l'écran pour signaler que l'on a atteint une phase de stabilisation.

Pendant ce palier, les quatre boutons triangulaires ont un effet s'ils sont pressés, alors qu'ils sont sans effet lors des périodes d'augmentation de l'intensité préprogrammées. Ainsi, une paire de bouton est dédiée au canal 1, et l'autre au canal 2. Ces boutons permettent donc une adaptation limitée du courant appliqué.

Le bouton avec pointe vers le haut permet d'augmenter par exemple d'1 mA l'intensité du courant délivré dans le canal associé. Il est possible de presser plusieurs fois ce bouton, ce qui a pour effet d'augmenter à chaque fois l'intensité de par exemple 1 mA. Si le bouton avec pointe vers le bas est pressé, le courant diminue de par exemple 1 mA. Il peut être utilisé par exemple 3 fois et le courant est à chaque fois diminué de par exemple 1 mA. À la par exemple quatrième utilisation, aucune action n'est déclenchée. À noter que lors des phases suivantes, l'effet de l'action sur un bouton avec pointe en bas sera limitée à une seule diminution d'intensité de par exemple 1 mA par phase. Ces boutons permettent donc une adaptation limitée du courant, dans les limites permettant de conserver l'efficacité du traitement.

De préférence, au bout de par exemple 4 minutes 20 secondes, une information est émise et/ou visualisée.

Un bip sonore retentit ensuite chaque seconde entre par exemple 4 min 55 et 5 min pour prévenir le patient d'une nouvelle augmentation de l'intensité à la par exemple cinquième minute.

Une pression du bouton orange de stabilisation permet alors d'empêcher l'augmentation de l'intensité et de la maintenir à sa valeur actuelle.

Il est important de noter que pour l'ensemble des phases d'utilisation du dispositif, le bouton orange de maintien de l'intensité ne modifie pas le reste du profil prédéterminé, ni l'augmentation de l'intensité lors des phases ultérieures du traitement. Il a donc pour seul effet de stabiliser ponctuellement l'intensité.

La phase d'augmentation du courant est alors répétée comme sur la figure 3, pendant un temps approximatif de par exemple 30 minutes (par exemple 6 phases), l'utilisateur ayant toujours la possibilité de maintenir le courant dans sa valeur actuelle avant les phases de croissance de l'intensité grâce au bouton orange, de préférence, par exemple, dans les 5 secondes précédent la croissance s'accompagnant d'un signal sonore.

À la fin de la dernière phase (par exemple phase 6), le courant est automatiquement remis à 0, et une phrase est émise et/ou visualisée signalant la fin du traitement.

Pendant l'ensemble des phases, l'utilisateur peut arrêter l'application du courant en pressant le bouton rouge de remise à 0.

Si le bouton vert est pressé, le programme de traitement est réinitialisé et commence au début de la phase 1 décrite plus haut.

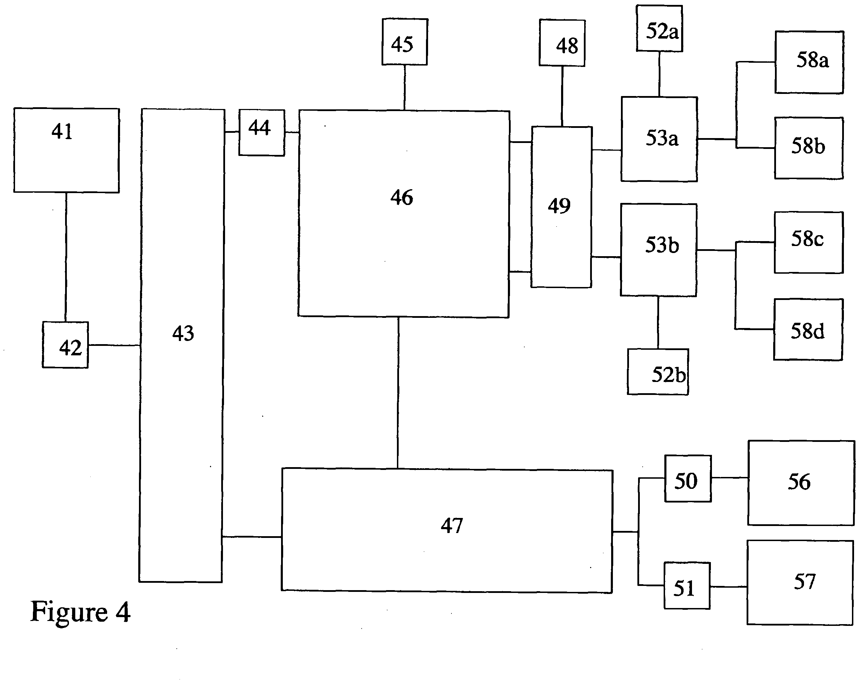

Afin de réaliser ces fonctionnalités de contrôle d'un profil prédéterminé par l'utilisateur, l'invention est illustrée figure 4. Selon cette figure, le dispositif de l'invention comprend une batterie ou une pile 41, un interrupteur général 42 et une alimentation 43. Il comprend par ailleurs un bouton de déclenchement ou de démarrage (bouton vert 45) et un bouton d'arrêt (bouton rouge 44). Ces deux boutons commandent le module générateur de profil de courant 46. Le bouton limiteur (bouton de stabilisation orange 48) contrôle quant à lui un limiteur de courant 49. Le courant est également réglable par des modules de régulation 53a et 53b commandées par des boutons de réglage manuel (boutons à flèches 52a et 52b). Ces deux canaux contrôlent alors les 4 électrodes de traitement à appliquer sur la peau du patient 58a,b,c,d.

Le générateur de profil de courant 46 est également connecté à un module d'informations pour l'utilisateur 47, commandé par des boutons de réglage 50 et 51. Les informations destinées à l'utilisateur sont alors produites sur l'écran 56 et par les écouteurs 57.

L'invention est décrite dans ce qui précède à titre d'exemple. Il est entendu que l'homme du métier est à même de réaliser différentes variantes de l'invention sans pour autant sortir du cadre du brevet.

## Revendications

1. Dispositif d'électrothérapie comprenant au moins une électrode (1) pour l'application d'un courant de traitement à un patient et un moyen de contrôle (6) comprenant au moins un moyen de déclenchement (9) et agencé pour initier la délivrance d'un profil de courant de traitement prédéterminé dans les différents paramètres dudit courant, notamment l'intensité, **caractérisé en ce que** ledit moyen de contrôle (6) comporte en outre des moyens agencés pour modifier le profil du courant de traitement appliqué en fonction du seuil de douleur du patient, lesdits moyens comprenant un moyen d'adaptation limitée dudit courant (12, 13) manipulable par un utilisateur et un moyen de stabilisation (10) agencé pour être activé, ledit moyen de stabilisation, lorsqu'il est activé, provoquant une modification dudit profil de courant de traitement en limitant l'intensité du courant à sa valeur au moment de l'activation dudit moyen de stabilisation (10).

2. Dispositif d'électrothérapie selon la revendication 1, **caractérisé en ce que** ledit moyen de contrôle (6) comprend un écran d'affichage (14).

3. Dispositif d'électrothérapie selon la revendication 1, **caractérisé en ce que** ledit moyen de contrôle (6) comprend au moins une sortie audio.

4. Dispositif d'électrothérapie selon la revendication 1, **caractérisé en ce que** ledit moyen de contrôle (6) comprend un moyen de remise à zéro (11) du courant.

5. Dispositif d'électrothérapie selon la revendication 1, **caractérisé en ce qu'**il comprend un moyen de contention (2, 3) pour ladite au moins une électrode (1).

6. Dispositif d'électrothérapie selon la revendication 5, **caractérisé en ce que** ledit moyen de contention (2, 3) est réglable au moyen d'au moins un ensemble de manchons coulissants (4a, 5a).

7. Dispositif d'électrothérapie selon la revendication 5, **caractérisé en ce que** ledit moyen de contention (2, 3) est adaptable à l'utilisation spécifique du dispositif en fonction de la pathologie à traiter et des différences anatomiques du patient.

## Claims

1. Electrotherapy device comprising at least one electrode (1) for applying a treatment current to a patient and one control means (6) comprising at least one triggering means (9) and arranged to initiate the delivery of a predetermined treatment-current profile in the various parameters of the said current, notably the intensity, **characterized in that** the said control means (6) also comprises means arranged to modify the profile of the treatment current applied as a function of the pain threshold of the patient, the said means comprising a means for limited adaptation of the said current (12, 13) that can be manipulated by a user and a stabilization means (10) arranged in order to be activated, the said stabilization means, when it is activated, causing a modification of the said treatment-current profile by limiting the intensity of the current to its value at the time of activation of the said stabilization means (10).

2. Electrotherapy device according to Claim 1, **characterized in that** the said control means (6) comprises a display screen (14).

3. Electrotherapy device according to Claim 1, **characterized in that** the said control means (6) comprises at least one audio output.

4. Electrotherapy device according to Claim 1, **characterized in that** the said control means (6) comprises a reset means (11) for resetting the current to zero.

5. Electrotherapy device according to Claim 1, **characterized in that** it comprises a contention means (2, 3) for the said at least one electrode (1).

6. Electrotherapy device according to Claim 5, **characterized in that** the said contention means (2, 3) can be adjusted using at least one set of sliding sleeves (4a, 5a).

7. Electrotherapy device according to Claim 5, **characterized in that** the said contention means (2, 3) can be adapted to the specific use of the device as a function of the pathology to be treated and of the anatomical differences of the patient.

## Patentansprüche

1. Elektrotherapiegerät, welches mindestens eine Elektrode (1) zum Aufbringen eines Behandlungsstromes auf einen Patienten und eine Regeleinrichtung (6) mit mindestens einer Auslösereinrichtung (9) aufweist, die angeordnet ist, um die Ausgabe eines zuvor in den unterschiedlichen Parametern festgelegten Behandlungsstromprofils zu initiieren, insbesondere demjenigen der Intensität,
**dadurch gekennzeichnet, dass** die Regeleinrichtung (6) außerdem Einrichtungen zum Verändern des angewandten Behandlungsstromprofils in Abhängigkeit von dem Schmerzschwellenwert des Patienten aufweist, wobei die Einrichtungen eine Einrichtung zur begrenzten Anpassung des Stromes (12,13), die von einem Benutzer handhabbar ist, sowie eine Stabilisierungseinrichtung (10) umfassen, die angeordnet ist, um aktiviert zu werden, wobei die Stabilisierungseinrichtung dann, wenn sie aktiviert wird, eine Veränderung des Behandlungsstromprofils herbeiführt, indem die Stärke des Stromes auf seinen Wert zum Zeitpunkt der Aktivierung der Stabilisierungseinrichtung (10) begrenzt wird.

2. Elektrotherapievorrichtung nach Anspruch 1,
**dadurch gekennzeichnet, dass** die Regeleinrichtung (6) einen Anzeigebildschirm (14) aufweist.

3. Elektrotherapievorrichtung nach Anspruch 1,
**dadurch gekennzeichnet, dass** die Regeleinrichtung (6) mindestens einen Audioausgang aufweist.

4. Elektrotherapievorrichtung nach Anspruch 1,
**dadurch gekennzeichnet, dass** die Regeleinrichtung (6) eine Einrichtung (11) zur Rückstellung des Stromes auf Null aufweist.

5. Elektrotherapievorrichtung nach Anspruch 1,
**dadurch gekennzeichnet, dass** sie eine Stützeinrichtung (2, 3) für die mindestens eine Elektrode (1) aufweist.

6. Elektrotherapievorrichtung nach Anspruch 1,
**dadurch gekennzeichnet, dass** die Stützeinrichtung (2, 3) mittels mindestens einer Gruppe von Gleithülsen (4a, 5a) regulierbar ist.

7. Elektrotherapievorrichtung nach Anspruch 5,
**dadurch gekennzeichnet, dass** die Stützeinrichtung (2, 3) auf die spezifische Verwendung der Vorrichtung in Abhängigkeit von der zu behandelnden Pathologie und den anatomischen Unterschieden des Patienten anpassbar ist.
